# EUROPEAN PATENT APPLICATION

(11) **EP 3 721 806 A1**
(43) Date of publication of application: **14.10.2020**
(21) Application number: 18885064.8
(22) Date of filing: 07.12.2018
(51) Int. Cl.: A61B 5/1455

(54) **RING-TYPE PULSE OXIMETER**

(30) Priority: 08.12.2017 CN 201721700222 U
(71) Applicant: Hangzhou Megasens Technologies Co., Ltd., Hangzhou, Zhejiang 310052 (CN)
(72) Inventor: YUAN, Haiquan, Zhejiang 310052 (CN); ZHOU, Congcong, Zhejiang 310052 (CN)
(74) Representative: Santarelli
(86) International application number: PCT/CN2018/119831
(87) International publication number: WO 2019/110010

(57) **Abstract**

Disclosed is a ring-type pulse oximeter, including a rigid ring body (1). A measurement unit (2) is arranged inside the rigid ring body (1), and an elastic device (3), a photodiode (4), and at least one light emitting diode (5) are protrudingly disposed on an inner circumferential surface of the rigid ring body (1). When the ring-type pulse oximeter is worn, the elastic device (3) is pressed, so that the photodiode (4) and the at least one light emitting diode (5) fit with a finger, light emitted by the light emitting diode (5) is attenuated by the finger, then received by the photodiode (4) and processed by the measurement unit (2) to calculate blood oxygen saturation. The ring-type pulse oximeter exerts a force on a portion of the finger, such that the finger maintains a tight fit to the photodiode (4) and the light-emitting diode (5), thereby providing a comfortable wearing experience as well as adaptability to different finger shapes, and improving measurement accuracy.

## Description

### TECHNICAL FIELD

The utility model relates to the medical device field, and in particular, to a ring-type pulse oximeter.

### BACKGROUND

Blood oxygen saturation is one of the key clinical physiological parameters. Currently, a monitor with a measurement host separated from a photoelectric sensor or a handheld blood oxygen saturation measurement instrument is mainly used for long term continuous measurement. These instruments are relatively large and are not suitable for wearing to perform continuous measurement. To make the blood oxygen saturation measurement instrument easy to wear, and not easy to fall off, there is a ring-type blood oxygen saturation measurement instrument worn on a finger in the prior art which solves the problem that the finger-clip type blood oxygen saturation measurement instrument is easy to fall off. However, because the elastic finger sleeve or the locking strap is used to adapt to different sizes of fingers, the problem of over-tightening is easily caused, and the radial pressure is exerted around the finger. Consequently, when the instrument is worn for a long time, it can cause poor blood flow and swelling feeling, which affects wearing comfort. In addition, since the shapes of the fingers are not the same, the fingers may not closely fit with the photoelectric sensor. Consequently, inaccurate measurement is caused.

### SUMMARY

The objective of the utility model is to provide a ring-type pulse oximeter. A finger is locally stressed, and closely fits with a photoelectric sensor, so that wearing comfort and adaptability to different finger shapes can be implemented, and measurement accuracy can be improved.

To solve the above technical problem, embodiments of the utility model disclose a ring-type pulse oximeter, including a rigid ring body. A measurement unit is arranged inside the rigid ring body. An elastic device, a photodiode, and at least one light emitting diode are protrudingly disposed on an inner circumferential surface of the rigid ring body. When the ring-type pulse oximeter is worn, the elastic device is pressed, so that the photodiode and the at least one light emitting diode fit with a finger, light emitted by the light emitting diode is attenuated by the finger, then received by the photodiode and processed by the measurement unit to calculate blood oxygen saturation.

In an example, the elastic device includes an elastic member and at least one layer of pressing member. The elastic member deforms when the elastic member is pressed,, and one end of the elastic device is connected to the rigid ring body, and the other end of the elastic device is connected to the lowest layer of the pressing member. When the elastic device includes multiple layers of pressing members, an upper-layer pressing member of adjacent two layers of pressing members is in an original state in which the upper-layer pressing member is partially embedded in a lower-layer pressing member when the elastic device is not pressed, the upper-layer pressing member moves towards the inside of the lower-layer pressing member when the elastic device is pressed, and after the pressing disappears, the upper-layer pressing member moves towards the outside of the lower-layer pressing member under an acting force of the elastic member and is restored to the original state.

In an example, the elastic member is a spring or a spring plate.

In an example, the pressing member is a hollow casing.

In an example, the elastic device, the photodiode, and the at least one light emitting diode are located on the same side of the rigid ring body.

In an example, a pressed surface of the uppermost-layer pressing member of the elastic device has a transparent portion, and the photodiode is located inside the elastic device and fits with the transparent portion.

In an example, the ring-type pulse oximeter includes two light emitting diodes located on two sides of the elastic device.

In an example, the photodiode and the at least one light emitting diode are respectively located on two sides of the elastic device.

In an example, the photodiode and the at least one light emitting diode are located on the same side of the rigid ring body, and the elastic device is located on the other side of the rigid ring body.

In an example, the elastic device includes a casing and a silica gel. The silica gel is fastened inside the casing and protrudes from a top end of the casing. When the silica gel is pressed, the silica gel deforms and moves towards the inside of the casing, and is restored to the protruding state after the pressing disappears.

In an example, the elastic device, the photodiode, and the at least one light emitting diode are located on the same side of the rigid ring body.

In an example, the photodiode is located inside the elastic device, and fits with the silica gel.

In an example, the ring-type pulse oximeter includes two photodiodes located on two sides of the elastic device.

In an example, the photodiode and at least one light emitting diode are respectively located on two sides of the elastic device.

In an example, the photodiode and the at least one light emitting diode are located on the same side of the rigid ring body, and the elastic device is located on the other side of the rigid ring body.

Comparing the embodiments of the utility model with the prior art, the main differences and effects are as follows: A protruding elastic device, a photodiode, and a light-emitting diode are protrudingly disposed on a rigid ring body, so that a finger is locally stressed to avoid poor blood flow, and implement wearing comfort and adaptability to different finger shapes. In addition, when the elastic device is pressed, the finger closely fits with the photodiode and the light emitting diode by a radial force that the elastic device exerts on the finger, so as to improve measurement accuracy.

### BRIEF DESCRIPTION OF DRAWINGS

FIG. 1 is a schematic diagram of an overall structure of a ring-type pulse oximeter according to an embodiment of the utility model;
FIG. 2 is a schematic structural diagram in which an elastic device, a photodiode, and a light emitting light are located on the same side according to an embodiment of the utility model;
FIG. 3 is a schematic structural diagram of an elastic device according to an embodiment of the utility model;
FIG. 4 is another schematic structural diagram of an elastic device according to an embodiment of the utility model; and
FIG. 5 is a schematic diagram in which an elastic device, a photodiode, and a light emitting diode are located in different sides according to an embodiment of the utility model.

### DESCRIPTION OF EMBODIMENTS

In the following description, numerous technical details are provided for a reader to better understand the present application. However, persons of ordinary skill in the art can understand that the technical solutions that the claims of the present application request to protect can be implemented without these technical details and various changes and modifications based on the following embodiments.

To make the objectives, technical solutions, and advantages of the embodiments of the present invention clearer, the following describes the embodiments of the present invention in detail with reference to the accompanying drawings.

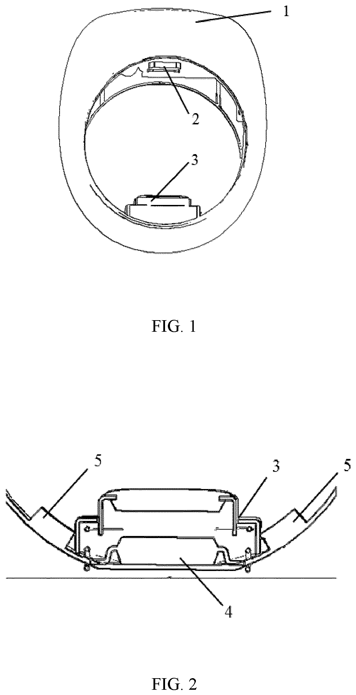

As shown in FIG. 1, a first embodiment of the utility model relates to a ring-type pulse oximeter, including a rigid ring body 1. A measurement unit 2 for measuring blood oxygen saturation is arranged inside the rigid ring body. An elastic device 3, a photodiode, and at least one light emitting diode are protrudingly disposed on an inner circumferential surface of the rigid ring body. When the ring-type pulse oximeter is worn, the elastic device is pressed, so that the photodiode and the at least one light emitting diode fit with a finger. The fitting means that the photodiode and the light emitting diode are closely adjacent to the finger and even in direct contact with the finger. The rigid ring body can be made of materials such as ceramic and metal. The measurement unit includes a power supply and its switch, a microprocessor, a light emitting drive circuit and the like to implement measurement of blood oxygen saturation, which can be implemented by using the prior art. There may be one or more light emitting diodes, and the photodiode, the light emitting diode, and the elastic device can be located on the same side (an upper side, a lower side, a left side, or a right side) of the rigid ring body, or can be located on different sides.

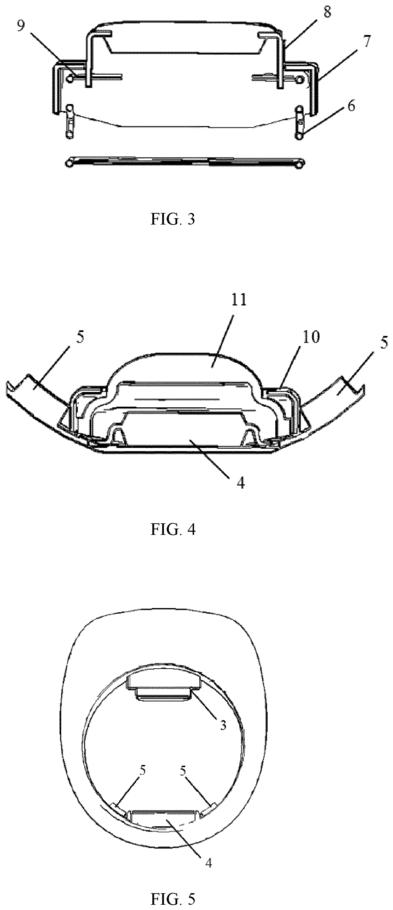

FIG. 2 is a structural diagram in which a photodiode 4, a light emitting diode 5, and an elastic device 3 are located on the same side of a rigid ring body. The photodiode 4 is located inside the elastic device 3, and fits with a pressed surface of the elastic device. Two photodiodes 5 are located on two sides of the elastic device 3. A structure of the elastic device 3 is shown in FIG. 3, and includes an elastic member 6 and two layers of pressing members 7 and 8, for example, hollow casings. One end of the elastic member 6 is connected to the rigid ring body 1, and the other end of the elastic member 6 is connected to the lower-layer pressing member 7. A surface of the upper-layer pressing member 8 that is pressed by a finger has a transparent portion. When the oximeter is not worn, and the elastic device 3 is not pressed, the upper-layer pressing member 8 is in an original state in which the upper-layer pressing member 8 is partially embedded in the lower-layer pressing member 7. When the oximeter is worn, and the elastic device 3 is pressed by the finger, the upper-layer pressing member 8 moves towards the inside of the lower-layer pressing member 7, and the elastic member 6 deforms simultaneously, so that the finger is in close contact with the light emitting diode 5, and this state is maintained; light emitted by the light emitting diode 5 is attenuated by the finger, then received by the photodiode 4 that fits with the transparent portion of the pressed surface of the upper-layer pressing member 8; further, the measurement unit 2 calculates blood oxygen saturation. When the oximeter is removed, the elastic device 6 is restored to the original shape. Under an acting force generated by the elastic device 6, the upper-layer pressing member 8 is ejected towards the outside of the lower-layer pressing member 7. To avoid that the upper-layer pressing member 8 is completely ejected from the lower-layer pressing member 7 and restore to the original state, a position limitation member 9 may be disposed in the lower-layer pressing member 7.

Another structure of the elastic device 3 is shown in FIG. 4, and includes a casing 10 and a silica gel 11. The silica gel 11 is fastened in the casing 11, and protrudes from the top end of the casing 8. When the oximeter is worn, a finger presses the silica gel 11, so that the silica gel 11 moves towards the inside of the casing 10 and deforms. In this way, the finger is in close contact with the light emitting diode 5, and this state is maintained; light emitted by the light emitting diode 5 is attenuated by the finger, then received by the photodiode 4 closely fitting with the silica gel 11, further the measurement unit 2 calculates blood oxygen saturation. When the oximeter is removed, the silica gel 11 is restored to the original shape.

In the above embodiments, when the oximeter is worn, the elastic device is pressed, so that the finger is in close contact with the light emitting diode 5. The relative rotation between the finger and the rigid ring body may be avoided by the radial pressure exerted on the finger due to deformation of the elastic member 6 or the silicone member 11, so that the finger is in close contact with the light-emitting diode 5. In addition, the photodiode 4 closely fit with the pressed surface of the elastic device. Therefore, the photodiode also closely fits with the finger. Measurement accuracy is improved based on the above two aspects. The elastic device 3, the photodiode 4, and the light-emitting diode 5 that are protrudingly disposed make the finger locally stressed, so that the oximeter can adapt to different shapes of fingers, can also avoid the swelling feeling caused by poor blood flow, and is more comfortable to wear. To adapt to fingers with different size, in the elastic device, there is a certain amount of retraction when the pressing member or the silica gel is pressed. However, regardless of the amount of retraction, the finger and the light emitting diode are in close contact, otherwise, a rigid ring body with another size needs to be chosen.

In addition, in the above embodiments, other optional settings may be made. For example, there may be a single layer of pressing member or multiple layers of pressing members in the elastic device. The single layer of pressing member is connected to one end of the elastic member, and a pressed surface of the single layer of pressing member has a transparent portion. The structure of the multiple layers of pressing members is the same as that of the two layers of pressing members, the bottommost-layer pressing member is connected to one end of the elastic member, and the pressed surface of the uppermost-layer pressing member has a transparent portion. The elastic member in the elastic device may be a spring or a spring plate made of metal or the like. There may be one or more light emitting diodes. The photodiode may be located outside the elastic device, and the photodiode and the light emitting diode are separately located on two sides of the elastic device. In this case, the pressed surface of the elastic device may not have a transparent portion. When the oximeter is worn, the finger presses the elastic device, and the elastic device deforms, so that the finger is in close contact with the light emitting diode and the photodiode, and this state is maintained.

FIG. 5 is a structural diagram in which a photodiode 4 and a light emitting diode 5 are located on the same side of a rigid ring body and an elastic device 3 is located on the other side. Preferably, if the photodiode and the light-emitting diode are viewed as an entirety, the entirety and the elastic device are respectively disposed at two ends of the diameter of the rigid ring body. There may be one or more light emitting diodes. The elastic device may adopt any one type in the above descriptions, and the pressed surface of the elastic device may not have a transparent portion. When the oximeter is worn, a finger is in contact with the light emitting diode and the photodiode, and presses the elastic device, the radial pressure exerted by the elastic device on the finger due to the deformation makes the contact between the finger and the light emitting diode and the photodiode closer, and this state is maintained, so that measurement accuracy is improved. The elastic device 3, the photodiode 4, and the light-emitting diode 5 that are protrudingly disposed make the finger locally stressed, so that the oximeter can adapt to different shapes of fingers, can also avoid the swelling feeling caused by poor blood flow, and is more comfortable to wear. To adapt to fingers of different size, in the elastic device, there is a certain amount of retraction when the pressing member or the silica gel is pressed. However, regardless of the amount of retraction, the finger is in close contact with the light emitting diode and the photodiode, otherwise, a rigid ring body of another size needs to be selected.

It should be noted that, in the claims and the description of the present patent, relational terms "first", "second", and the like are only used to distinguish one entity or operation from another entity or operation, but are not intended to indicate any actual relationship or order between entities or operations. Moreover, the terms "include", "have", and any other variants thereof are intended to cover non-exclusive inclusion, for example, a process, a method, article, or a device that includes a series of elements not only includes those elements, but includes other elements that are not clearly listed or are inherent to the process, the method, the article, or the device. Without more restrictions, an element that is defined by the phrase "including a" does not exclude the presence of another same element in the process, method, article, or device that includes the element.

Although the present invention has been illustrated and described with reference to the preferred embodiments of the present invention, it should be understood by persons of ordinary skills in the art that various changes in form and details may be made without departing from the spirit and scope of the present invention.

## Claims

1. A ring-type pulse oximeter, comprising a rigid ring body (1), wherein a measurement unit (2) is arranged inside the rigid ring body (1), an elastic device (3), a photodiode (4), and at least one light emitting diode (5) are protrudingly disposed on an inner circumferential surface of the rigid ring body (1), and when the ring-type pulse oximeter is worn, the elastic device (3) is pressed, so that the photodiode (4) and the at least one light emitting diode (5) fit with a finger, light emitted by the light emitting diode (5) is attenuated by the finger, then received by the photodiode (4) and processed by the measurement unit (2) to calculate blood oxygen saturation.

2. The ring-type pulse oximeter according to claim 1, wherein the elastic device (3) comprises an elastic member (6) and at least one layer of pressing member; the elastic member (6) deforms when the elastic device is pressed, one end of the elastic device is connected to the rigid ring body (1), and the other end of the elastic device is connected to the lowest layer of the pressing member, and when the elastic device (3) comprises multiple layers of pressing members, an upper-layer pressing member of adjacent two layers of pressing members is in an original state in which an upper-layer pressing member is partially embedded in the lower-layer pressing member when the elastic device (3) is not pressed, and the upper-layer pressing member moves towards the inside of the lower-layer pressing member when the elastic device (3) is pressed, and after the pressing disappears, the upper-layer pressing member moves towards the outside of the lower-layer pressing member under an acting force of the elastic member (6) and is restored to the original state.

3. The ring-type pulse oximeter according to claim 2, wherein the elastic member is a spring or a spring plate.

4. The ring-type pulse oximeter according to claim 2, wherein the pressing member is a hollow casing.

5. The ring-type pulse oximeter according to any one of claims 2-4, wherein the elastic device (3), the photodiode (4), and the at least one light emitting diode (5) are located on the same side of the rigid ring body (1).

6. The ring-type pulse oximeter according to claim 5, wherein a pressed surface of the uppermost-layer pressing member of the elastic device (3) has a transparent portion, and the photodiode (4) is located inside the elastic device (3) and fits with the transparent portion.

7. The ring-type pulse oximeter according to claim 6, wherein, comprising two light emitting diodes (5) located on two sides of the elastic device (3).

8. The ring-type pulse oximeter according to claim 5, wherein the photodiode (4) and the at least one light emitting diode (5) are respectively located on two sides of the elastic device (3).

9. The ring-type pulse oximeter according to any one of claims 2-4, wherein the photodiode (4) and the at least one light emitting diode (5) are located on the same side of the rigid ring body (1), and the elastic device (3) is located on the other side of the rigid ring body (1).

10. The ring-type pulse oximeter according to claim 1, wherein the elastic device comprises a casing (10) and a silica gel (11), wherein the silica gel (11) is fastened inside the casing (10) and protrudes from a top end of the casing (10), when the silica gel (11) is pressed, the silica gel (11) deforms and moves towards the inside of the casing (10), and is restored to the protruding state after the pressing disappears.

11. The ring-type pulse oximeter according to claim 10, wherein the elastic device (3), the photodiode (4), and the at least one light emitting diode (5) are located on the same side of the rigid ring body (1).

12. The ring-type pulse oximeter according to claim 11, wherein the photodiode (4) is located inside the elastic device (3), and fits with the silica gel (11).

13. The ring-type pulse oximeter according to claim 12, wherein, comprising two photodiodes (5) located on two sides of the elastic device (3).

14. The ring-type pulse oximeter according to claim 11, wherein the photodiode (4) and at least one light emitting diode (5) are respectively located on two sides of the elastic device (3).

15. The ring-type pulse oximeter according to claim 10, wherein the photodiode (14) and the at least one light emitting diode (5) are located on the same side of the rigid ring body (1), and the elastic device (3) is located on the other side of the rigid ring body (1).
